Europäisches Patentamt

European Patent Office

Office européen des brevets

(19)

(11) Numéro de publication: **0 001 199**
**B1**

(12) **FASCICULE DE BREVET EUROPEEN**

(45) Date de publication du fascicule de brevet: **16.09.81**

(21) Numéro de dépôt: **78420006.5**

(22) Date de dépôt: **30.08.78**

(51) Int. Cl.³: **C 07 C 50/04,**
C 07 C 45/38, B 01 J 23/70

(54) Procédé de préparation de para-benzoquinone.

(30) Priorité: **05.09.77 FR 7727530**

(43) Date de publication de la demande:
**21.03.79 Bulletin 79/6**

(45) Mention de la délivrance du brevet:
**16.09.81 Bulletin 81/37**

(84) Etats Contractants Désignés:
**BE CH DE GB LU NL SE**

(56) Documents cités:
FR - A - 2 138 030
US - A - 3 213 114

(73) Titulaire: **RHONE-POULENC INDUSTRIES
22, avenue Montaigne
F-75008 Paris (FR)**

(72) Inventeur: **Costantini, Michel
9, place Aristide Briand
F-69003 Lyon (FR)**
Inventeur: **Jouffret, Michel
Le Grillon Avenue du Chater
F-69340 Francheville-Le-Bas (FR)**

(74) Mandataire: **Rioufrays, Roger et al,
RHONE-POULENC INDUSTRIES Centre de
Recherches des Carrières Service Brevets
F-69190 Saint-Fons (FR)**

Procédé de préparation de para-benzoquinone.

La présente invention a pour objet un procédé de préparation de la para-benzoquinone par oxydation du phénol par l'oxygène ou un gaz en contenant.

La para-benzoquinone-que l'on désignera plus simplement ci-après benzoquinone- est un produit organique particulièrement important du point du vue industriel puisqu'il permet d'accéder par hydrogénation à l'hydroquinone utilisée notamment dans l'industrie photographique.

On a proposé de nombreux procédés de préparation de l'hydroquinone à partir du phénol; ces procédés mettent en jeu l'hydroxylation du phénol essentiellement par l'eau oxygénée elle-même ou par des peracides organiques tels que les acides peracétique et performique engendrés "in situ" à partir de l'eau oxygénée et de l'acide carboxylique. Ils conduisent dans tous les cas à la formation con-comittante d'hydroquinone et de pyrocatéchine, ce dernier produit étant généralement formé en quantités prépondérantes. Certains de ces procédés d'hydroxylation par l'eau oxygénée se sont révélés d'un grand intérêt et font l'objet d'une exploitation industrielle pour la production des diphénols. Néanmoins l'industrie est à la recherche d'un procédé permettant d'obtenir sélectivement l'hydroquinone à partir du phénol, la formation des autres diphénols et notamment de la pyrocatéchine étant limitée voir même totalement évitée. L'oxydation sélective du phénol en para-benzoquinone apparaît comme un moyen de résoudre le problème et c'est pourquoi on assite à un effort de recherche de l'industrie pour la mise au point d'un procédé d'oxydation du phénol en benzoquinone par l'oxygène moléculaire ou un gaz en contenant.

Ainsi, dans la demande de brevet français publiée sous le numéro 2 245 602, on a décrit un procédé de préparation de benzoquinone à partir de divers phénols et notamment de la benzoquinone à partir du phénol par oxydation par l'oxygène moléculaire ou un gaz qui en contient (air par exemple), en présence d'un catalyseur comprenant du cuivre et un ligand chlore, brome, iode, thiocyanate, cyanate ou cyanure, dans un solvant polaire. Bien que le cuivre métallique puisse être mis en oeuvre dans des conditions qui permettent son oxydation en ions cuivreux ou cuivriques, ce sont généralement des sels de cuivre qui sont utilisés et notamment des halogénures cuivreux ou cuivriques et en particulier le chlorure cuivrique. Dans le brevet américain 3 987 068 on a proposé un procédé analogue selon lequel la réaction est conduite en présence d'un sel de cuivre dans un nitrile formant un complexe avec le sel de cuivre. Bien que ces procédés assurent généralement un bon taux de conversion du phénol et des rendements industriellement intéressants en benzoquinone, on a constaté que lorsque l'on opère dans un appareillage qui n'est pas inerte vis-à-vis du milieu réactionnel -appareil en acier ou en fer- ses parois sont rapidement attaquées à un degré tel qu'il est pratiquement impossible d'envisager la mise en oeuvre industrielle d'un tel procédé. D'autre par ton a constaté que si l'on utilise un appareillage inerte vis-à-vis du milieu réactionnel la réaction n'a pratiquement pas lieu lorsqu'on la conduit en présence d'ions cuivriques; le recours à la présence d'un agent complexant du cuivre tel que ceux précités est nécessaire pour obtenir la formation de benzoquinone, mais le taux de transformation du phénol et les rendements en benzoquinone restent limités. Dans la demande française FR 2 138 030, on a décrit un procédé d'oxydation de phénols substitués en les quinones substituées correspondantes par l'oxygène en présence d'ions cuivre, d'ions halogènes et d'un agent complexant des ions métalliques, au sein d'un solvant qui peut être indifféremment un alcool, un glycol, un éther ou un amide. Les ions cuivriques sont préférés pour la mise en oeuvre de ce procédé et quel que soit le solvant utilisé les résultats obtenus sont du même ordre de grandeur. En définitive, il résulte de l'enseignement de la demande française FR 2 138 030 que lors de l'oxydation des phénols substitués, il est préférable d'utiliser des ions cuivriques et que la nature du solvant présent est sans effet sur le déroulement de la réaction. La présente invention concerne un procédé d'oxydation du phénol en benzoquinone dans un appareillage inerte vis-à-vis du milieu réactionnel et qui procure des taux de transformation améliorés du phénol et/ou des rendements supérieurs en benzoquinone.

Plus spécifiquement la présente invention a pour objet un procédé de préparation de benzoquinone par oxydation du phénol par l'oxygène moléculaire ou un gaz qui en contient, dans un solvant organique, en présence d'ions cuivreux ou cuivriques et d'un agent complexant du cuivre, caractérisé en ce que l'on opère en présence d'un agent réducteur des ions cuivriques en ions cuivreux pris dans le groupe formé par les diphénols, les phénols alcoyl substitués, les aldéhydes aliphatiques, les cétones aliphatiques comportant de 3 à 6 atomes de carbone, les oléfines et dioléfines aliphatiques et cycloaliphatiques.

Comme exemple de phénols substitués utilisables comme réducteurs, on peut citer les crésols, les diméthylphénols, les triméthylphénols tels que le triméthyl-2,4,6 phénol, le triméthyl-2,3,6 phénol et le triméthyl-2,3,5 phénol; comme diphénols on peut citer l'hydroquinone et la triméthylhydroquinone; parmi les aldéhydes aliphatiques on fait appel de préférence aux alcanals inférieurs tels que le formaldéhyde, l'éthanal et le propanal; parmi les cétones on peut citer l'acétone, la méthyléthylcétone, la méthylisopropylcétone, la méthylisobutylcétone, l'oxyde de mésityle et parmi les oléfines et dioléfines, le cyclohexène et l'isoprène.

La quantité de réducteur mis en oeuvre peut varier dans de larges limites car il peut servir de milieu réactionnel lorsqu'il est liquide dans les conditions de la réaction. En général elle est d'au moins 0,01 mole de réducteur par ion cuivre présent, et de préférence d'au moins 0,1 mole par ion cuivre, et plus préférentiellement encore au moins 1 mole.

Lorsque l'on opére en présence d'un tiers solvant il n'est pas nécessaire d'utiliser plus de 2 moles de réducteur par ion cuivre.

Comme tiers solvant on utilise des composés organiques polaires pris dans le groupe formé par les nitriles, les alcools aliphatiques inférieurs (alcools ayant de 1 à 4 atomes de carbone), les amides d'amines secondaires et les sulfoxydes. Parmi ces composés on fait appel de préférence à l'acétonitrile et au méthanol. Lorsqu'on utilise un réducteur comme solvant on fait appel de préférence à l'acétone, la méthyléthylcétone, la méthylisobutylcétone.

Les agents complexants du cuivre sont choisis parim les composés introduisant dans le milieu réactionnel des anions cyanate, thiocyanate, cyanure et halogènure. On fait appel de préférence à des sels alcalins ou alcalino-terreux. Le chlorure, le bromure et le fluorure de lithium conviennent tout particulièrement bien. La quantité de ces sels est en général comprise entre 0,1 et 5 moles par ion cuivre et de préférence entre 0,5 et 2.

Comme source d'ions cuivre — I ou II, on peut utiliser divers sels de cuivre, toutefois on fait appel préférence aux halogénures de cuivre et en particulier aux chlorures cuivreux ou cuivriques et au nitrate cuivrique.

La quantité de catalyseur au cuivre exprimé en nombre d'ions cuivre par mole de phénol peut varier dans de larges limites. En général cette quantité représente de 0,01 à 5 ions cuivre par mole de phénol; cependant il n'est pas souhaitable d'un point de vue pratique d'avoir recours à des quantités de catalyseur introduisant plus de 1 ion cuivre par mole de phénol. On utilise en général de 0,02 à 1 ion cuivre par mole de phénol.

La température à laquelle on conduit la réaction peut varier dans de larges limites. Des températures de 10 à 120°C et de préférence de 20 à 100°C conviennent bien. L'oxydation est réalisée sous une pression partielle d'oxygène d'au moins 5 bars. Bien qu'il n'y ait pas de limite supérieure critique de la pression, en pratique, on ne recourt pas à des pressions partielles d'oxygène supérieures à 100 bars et de préférence supérieures à 50 bars.

Le gaz contenant de l'oxygène moléculaire peut être l'air ou de l'air appauvri ou enrichi en oxygène, ou des mélanges d'oxygène avec divers gaz inertes.

La concentration du phénol dans le solvant n'est pas critique et peut prendre des valeurs extrêmement variées.

D'un point de vue pratique, la réaction est conduite dans un appareillage résistant à la pression, inerte vis-à-vis de la masse réactionnelle tel que les autoclaves en acier émaillé ou chemisé en tantale.

Les exemples suivants illustrent l'invention et montrent comment elle peut être mise en pratique.

Exemple 1

Dans un autoclave chemisé en tantale de 0,5 l, agité par secousses, on charge successivement: 90 cm3 de méthanol; 0,075 mole de phénol; 0,022 mole de $CuCl_2$; 0,022 mole de propanal et 0,044 mole de chlorure de lithium. On ferme l'autoclave puis introduit de l'air jusqu'à une pression de 100 bars; après quoi on porte la température à 70°C. On maintient le contenu de l'appareil dans ces conditions pendant 2 heures sous agitation. Après refroidissement, on dégaze la masse réactionnelle dans laquelle on dose par chromatographie en phase gazeuse et par polarographie, le phénol non transformé, la p-benzoquinone et le cas échéant les diphénols.

Le taux de transformation du phénol s'élève dans ces conditions à 89% et le rendement en benzoquinone par rapport au phénol transformé à 60%.

A titre comparatif on a répété l'exemple précédent mais d'abord en opérant en absence de chlorure de lithium et de propanal (essai A), puis en opérant seulement en absence de propanal (essai B). Les résultats ont été les suivants:

| | Taux de transformation du phénol % | Rendement en benzoquinone % |
|---|---|---|
| Essai A | 0 | 0 |
| Essai B | 46 | 41,5 |

Exemples 2 à 8

On a opéré comme à l'exemple 1, à 65°C, en faisant varier la nature du réducteur, les autres conditions restant les mêmes, sauf indications contraires. Les résultats ont été rassemblés dans le tableau suivant:

**0 001 199**

| EXEMPLES | REDUCTEURS | | TAUX TRANSFORMATION % | RENDEMENTS BENZOQUINONE % |
| | NATURE | QUANTITE EN MOLES | | |
|---|---|---|---|---|
| 2 | hydroquinone | 0,033 | 77,5 | 47 |
| 3 | triméthyl-2,3,6 phénol | 0,033 | 98,5 | 50,5 |
| 4 | isopréne | 0,033 | 82,5 | 28 |
| 5 | méthyléthylcétone | (1) | 98,6 | 58 |
| 6 | cyclohexène | 0,033 | 64 | 53 |
| 7 | méthyléthylcétone | (2) | 93 | 59,5 |
| 8 | acétone | (3) | 88 | 55,5 |

(1) la quantité de méthyléthylcétone est de 75 cm3 et la quantité de méthanol de 15 cm3.

(2) exemple réalisé dans la méthyléthylcétone (90 cm3) sans tiers solvant, en présence de 0,022 mole de CuCl au lieu de $CuCl_2$.

(3) exemple réalisé comme l'exemple 7 mais dans 90 cm3 d'acétone.

### Exemple 9

On opère comme à l'exemple 8 mais en présence de $CuCl_2$ au lieu de CuCl. La masse réactionnelle est portée 2 heures à 70°C, puis laissée au repos 64 heures à 20°C. La taux de transformation du phénol s'élève à 81% et le rendement en para-benzoquinone à 45%.

### Exemples 10 à 13

On opère comme à l'exemple 8 dans les conditions suivantes:

solvant: acétone à 0,2% d'eau

concentration du phénol dans l'acétone: 0,83 M/l

température: 70°C

pression d'air: 100 bars

durée: 4 heures

catalyseur: chlorure cuivreux

en faisant varier le rapport molaire CuCl/phénol et le rapport molaire LiCl/CuCl, on a obtenu les résultats suivants:

| Exemples | CuCl/phénol | LiCl/CuCl | TT (1) phénol % | RT (2) % |
|---|---|---|---|---|
| 10 | 0,1 | 2 | 57 | 61 |
| 11 | 0,2 | 2 | 97 | 52 |
| 12 | 0.025 | 1 | 50,5 | 63 |
| 13 | 0,05 | 1 | 77,5 | 64,5 |

(1) Taux de transformation

(2) Rendement en benzoquinone par rapport au phénol transformé.

### Exemple 14

On opère comme à l'exemple 13 en remplaçant l'acétone par la méthylisobutylcétone. La taux de transformation du phénol s'élève à 65% et le rendement en benzoquinone par rapport au phénol transformé à 70,5%.

4

# 0 001 199

## Exemples 15 et 16

On opère comme à l'exemple 13 mais à 60°C dans l'acétone à 0,08% d'eau et la méthyléthylcétone à 0,016% d'eau.

On a obtenu les résultats suivants:

| EXEMPLES | CETONES | TT (1) % | RT (2) % |
|---|---|---|---|
| 15 | acétone | 47 | 80 |
| 16 | méthyléthylcétone | 59,5 | 80 |

(1) taux de transformation

(2) rendement en benzoquinone par rapport au phénol transformé.

## Revendications

1. Procédé de préparation de para-benzoquinone par oxydation du phénol par l'oxygène moléculaire ou un gaz qui en contient dans un solvant organique, en présence d'ions cuivreux ou cuivriques et d'un agent complexant du cuivre, caractérisé en ce que l'on opère en présence d'un agent réducteur des ions cuivriques en ions cuivreux pris dans le groupe formé par les diphénols, les phénols alcoyl substitués, les aldéhydes aliphatiques, les cétones aliphatiques ayant de 3 à 6 atomes de carbone, les oléfines et dioléfines aliphatiques et cycloaliphatiques.

2. Procédé selon la revendication 1, caractérisé en ce que le réducteur est pris dans le groupe formé par le triméthyl-2,3,6 phénol, l'hydroquinone, l'acétone, la méthylisobutylcétone, la méthyléthylcétone, l'oxyde de mésityle, l'isoprène, le cyclohexène.

3. Procédé selon la revendication 1, caractérisé en ce que la quantité de réducteur est d'au moins 0,01 mole par ion cuivre.

4. Procédé selon la revendication 1, caractérisé en ce que l'agent complexant du cuivre est un halogénure alcalin ou alcalino-terreux.

5. Procédé selon l'une quelconque des revendications 1 à 4, caractérisé en ce que la quantité d'agent complexant est comprise entre 0,1 et 5 moles par ion cuivre.

6. Procédé selon l'une quelconque des revendications 1 à 5, caractérisé en ce que le solvant est le méthanol et l'acétonitrile.

7. Procédé selon l'une quelconque des revendications 1 à 6, caractérisé en ce que la température est comprise entre 10 et 120°C et la pression partielle d'oxygène entre 5 et 100 bars.

8. Procédé selon l'une quelconque des revendications 1 à 7, caractérisé en ce que les ions cuivre sont sous forme de chlorure cuivrique.

9. Procédé selon l'une quelconque des revendications 1 à 8, caractérisé en ce que la quantité d'ions cuivre par mole de phénol est comprise entre 0,01 et 5.

10. Procédé selon l'une quelconque des revendications 1 à 9, caractérisé en ce que l'on opère dans un réducteur comme solvant.

11. Procédé selon la revendication 10, caractérisé en ce que le réducteur est l'acétone, la méthyléthylcétone, la méthylisobutylcétone.

## Patentansprüche

1. Verfahren zur Herstellung von para-Benzochinon durch Oxydation von Phenol mit molekularem Sauerstoff oder einem diesen enthaltenden Gas in einen organischen Lösungsmittel in Gegenwart von Kupfer-I oder -II-ionen und einem Komplexbildner für Kupfer, dadurch gekennzeichnet, daß man es in Gegenwart eines Reduktionsmittels der Kupfer-II-ionen in Kupfer-I-ionen, ausgewählt aus der durch Diphenole, alkylsubstituierte Phenole, aliphatische Aldehyde, aliphatische Ketone mit 3 bis 6 Kohlenstoffatomen, aliphatische und cycloaliphatische Olefine und Diolefine gebildeten Gruppe durchführt.

2. Verfahren nach Anspruch 1, dadurch gekennzeichnet, daß das Reduktionsmittel aus der durch 2,3,6-Trimethyl-phenol, Hydrochinon, Aceton, Methylisobutylketon, Methyläthylketon, Mesityloxid, Isopren und Cyclohexen gebildeten Gruppe genommen wird.

3. Verfahren nach Anspruch 1, dadurch gekennzeichnet, daß die Menge des Reduktionsmittels mindestens 0,01 Mol pro Kupferionbeträgt.

4. Verfahren nach Anspruch 1, dadurch gekennzeichnet, daß der Komplexbildner für Kupfer ein Alkali- oder Erdalkalihalogenid ist.

5. Verfahren nach einem der Ansprüche 1 bis 4, dadurch gekennzeichnet, daß die Menge des

5

Komplexbildners zwischen 0,1 und 5 Mol pro Kupferion beträgt.

6. Verfahren nach einem der Ansprüche 1 bis 5, dadurch gekennzeichnet, daß das Lösungsmittel Methanol und Acetonitril ist.

7. Verfahren nach einem der Ansprüche 1 bis 6, dadurch gekennzeichnet, daß die Temperatur zwischen 10 und 120°C und der Sauerstoffpartialdruck zwischen 5 und 100 bar beträgt.

8. Verfahren nach einem der Ansprüche 1 bis 7, dadurch gekennzeichnet, daß die Kupferionen in Form von Kupfer-II-chlorid vorliegen.

9. Verfahren nach einem der Ansprüche 1 bis 8, dadurch gekennzeichnet, daß die Menge an Kupferionen pro Mol Phenol zwischen 0,01 und 5 beträgt.

10. Verfahren nach einem der Ansprüche 1 bis 9, dadurch gekennzeichnet, daß man es in einem Reduktionsmittel als Lösungsmittel durchführt.

11. Verfahren nach Anspruch 10, dadurch gekennzeichnet, daß das Reduktionsmittel Aceton, Methyläthylketon, Methylisobutylketon ist.

## Claims

1. Process for the preparation of para-benzoquinone by oxidising phenol with molecular oxygen or with a gas containing molecular oxygen, in an organic solvent, in the presence of cuprous or cupric ions and of a copper complexing agent, characterised in that it is carried out in the presence of an agent for reducing cupric ions to cuprous ions, selected from the group consisting of the diphenols, the substituted alkylphenols, the aliphatic aldehydes, the aliphatic ketones containing from 3 to 6 carbon atoms, and the aliphatic and cycloaliphatic olefines and diolefines.

2. Process according to claim 1, characterised in that the reducing agent is selected from the group consisting of 2,3,6-trimethylphenol, hydroquinone, acetone, methyl isobutyl-ketone, methyl ethyl ketone, mesityl oxide, isoprene and cyclohexene.

3. Process according to claim 1, characterised in that the amount of reducing agent is at least 0.01 mol per copper ion.

4. Process according to claim 1, characterised in that the copper complexing agent is an alkali metal halide or alkaline earth metal halide.

5. Process according to any one of claims 1 to 4, characterised in that the amount of complexing agent is between 0.1 and 5 mols per copper ion.

6. Process according to any one of claims 1 to 5, characterised in that the solvent is methanol and acetonitrile.

7. Process according to any one of claims 1 to 6, characterised in that the temperature is between 10 and 120°C and the partial oxygen pressure is between 5 and 100 bars.

8. Process according to any one of claims 1 to 7, characterised in that the copper ions are in the form of cupric chloride.

9. Process according to any one of claims 1 to 8, characterised in that the amount of copper ions per mol of phenol is between 0.01 and 5.

10. Process according to any one of claims 1 to 9, characterised in that it is carried out in a reducing agent as the solvent.

11. Process according to claim 10, characterised in that the reducing agent is acetone, methyl ethyl ketone or methyl isobutyl ketone.